Europäisches Patentamt

(19) European Patent Office

Office européen des brevets

(11) Publication number: **0 144 213**
**A2**

(12) # EUROPEAN PATENT APPLICATION

(21) Application number: **84308291.8**

(22) Date of filing: **29.11.84**

(51) Int. Cl.⁴: **C 02 F 3/28**
**C 12 M 1/00**

(30) Priority: **30.11.83 GB 8331932**
**20.01.84 GB 8401521**

(43) Date of publication of application:
**12.06.85 Bulletin 85/24**

(84) Designated Contracting States:
**AT BE CH DE FR GB IT LI LU NL SE**

(71) Applicant: **CLEARFIELD N.V.**
**Schottegatweg Oost 130**
**Salinja Curacao Dutch Antilles(NL)**

(72) Inventor: **Kinahan, Edward Anthony Francis**
**Weatherside, Hound Corner Hamble Lane**
**Netley Abbey Southampton SO3 5FT(GB)**

(74) Representative: **Crampton, Keith John Allen et al,**
**D YOUNG & CO 10 Staple Inn**
**London WC1V 7RD(GB)**

(54) **Digester and its use.**

(57) Apparatus for anaerobic fermentation of animal waste to produce methane comprises an airtight vessel (1) having a paddle system (3) to break up crust on the surface and expel it via a valve (5, 6, 7) to atmosphere without admitting air. The paddle system (3) can be rotated by a Pelton wheel (10) operated by a steam of incoming slurry, the wheel (10) also serving to direct the stream to heat it near a heater (14) and to agitate the slurry in the vessel. Rotation of the paddle system (3) and wheel (10) takes place intermittently as slurry is admitted, and coincides with opening of the valve (5, 6, 7). A paddle disperses heated liquid from the heater (14) and gently mixes the liquid in the digester.

Fig.3.

EP 0 144 213 A2

1

# DIGESTER AND ITS USE

This invention relates to digesters for treating animal excreta to form methane gas. The methane-forming bacteria are anaerobic and therefore it is necessary to carry out such digestion in an airtight vessel. The mass of digested material is in the form of an aqueous slurry.

A problem that arises in this process is the formation of a solid crust on top of the slurry. This crust impedes the escape of methane and it is therefore necessary to break it up from time to time. Hitherto, this has involved opening the vessel to give access to the crust, with consequent admission of air, the presence of which results in the bacteria almost ceasing to function until an atmosphere of methane has built up again. In addition, of course, the admission of air results in the formation of an explosive mixture with the methane.

In accordance with the present invention, an airtight vessel having heating means at the bottom is equipped with one or more (preferably two) paddles mounted on a vertical shaft so that the paddles rotate in a horizontal plane, the height of the paddles being such as to coincide, in operation, with the top of the slurry of digesting material. The height of the paddles may be adjustable manually from outside or there may be some sort of float that keeps the paddles at the correct height.

In addition, an outlet means situated at the periphery of the vessel at the level of the paddles or just above, enables rotation of the shaft and hence of the paddles to break up the crust and push the solid material, together with any overflow of slurry resulting from the addition of further slurry to the vessel, through the outlet along a conduit to a receiver, from which it may flow out of the sealed system through a suitable weir, the pressure in the system, being about half an atmosphere in excess of atmospheric pressure, preventing any air from entering the system.

The outlet means may be a valve in the form of a vertical cylinder including a piston bearing a washer, the washer closing the outlet when in the lower position and opening it in the upper position. Other forms of valve may also be used, e.g. a sliding apertured member that can be moved so that the aperture coincides with an aperture in the wall in the open position and so that the member closes the aperture in the wall in the closed position.       —

The shaft carrying the paddles may be driven by means external to the digester vessel, e.g. an electric or hydraulic motor, but it is preferably driven by a turbine, e.g. a Pelton wheel, which is in a vertical plane when the vessel is upright and which is rotated by slurry entering through an opening near the bottom of the vessel.   The slurry enters the top of the Pelton wheel in a horizontal direction and leaves at the bottom in substantially the opposite direction, though preferably at a slight angle below the horizontal so as to come into close proximity to a heating dome situated in the centre of the bottom of the vessel.   Not only does the slurry passing into the Pelton wheel rotate the paddles but also the slurry leaving it, being turbulent as it escapes from the constriction of the Pelton wheel, has the effect of stirring up the slurry already in the body of the vessel, thereby keeping the digesting mixture agitated and heating the incoming slurry and mixing it with the slurry already in the vessel, thereby avoiding low temperatures, which adversely affect the rate at which the bacteria act.   It may be desirable for the Pelton wheel to carry on its shaft a disc or other indicator outside of the vessel so that observation can confirm whether or not the wheel is rotating.

There may be further means for agitating the liquid in the digester, e.g. a rotatable paddle well below the surface of the liquid.

The vessel has a closable conduit opening into the top space, preferably into the top surface, from which methane may be removed for collection.   The opening of this conduit will be controlled, possibly automatically by a pressure-sensitive device, depending on the gas pressure in the top of the vessel.

Preferably the introduction of fresh slurry is carried out intermittently.  This may be achieved by means of a pump for fresh slurry operated by a timing device, which causes the pump to operate for say ten minutes in every forty, and opens the valve during operation of the pump. Thus, during introduction of fresh slurry, the Pelton wheel is rotated and causes the paddles to expel the crust with overflowing slurry.

By using an apparatus in accordance with the invention, the crust can readily be removed without the need to open the vessel.   Obviously, the removal of crust can take place much more frequently and build-up of it thus be avoided. The particularly preferred apparatus, i.e. that having a suitably positioned Pelton wheel and an inlet pump with automatic timer, can be used to keep the fermenting slurry not only reasonably crust-free but also more uniform throughout with respect to temperature and to the presence of undissolved matter.

In the accompanying illustrative drawings,

Fig. 1 shows a horizontal section of a device in accordance with the invention, looking downwardly from the line I-I in Figs. 2 and 3;

Fig. 2 shows a vertical section through the device along line II-II in Fig 1;

Fig. 3 shows a vertical section through the device along line III-III in Fig. 1.

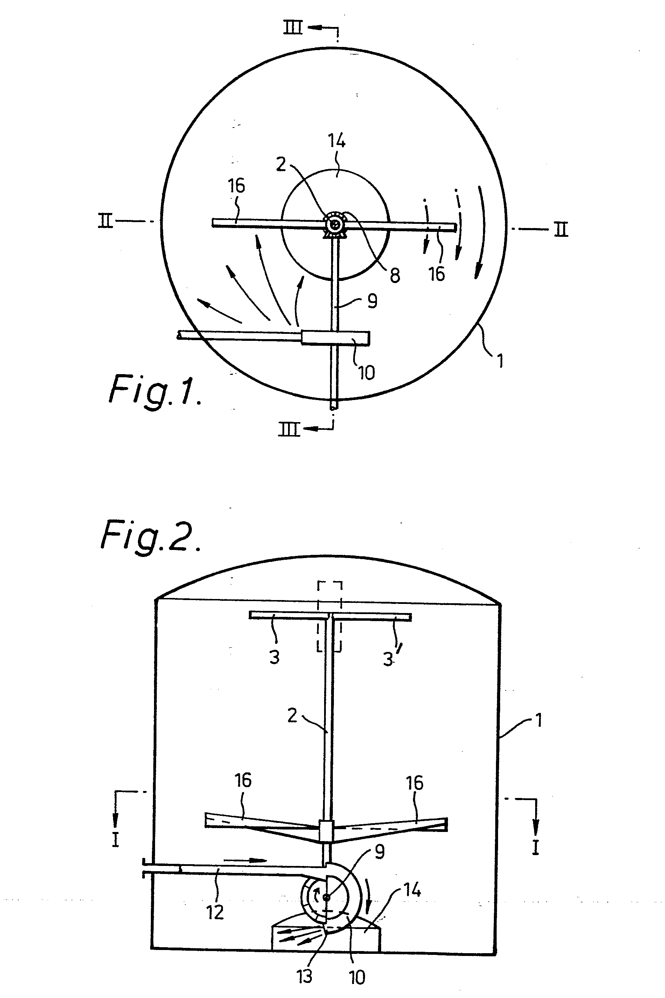

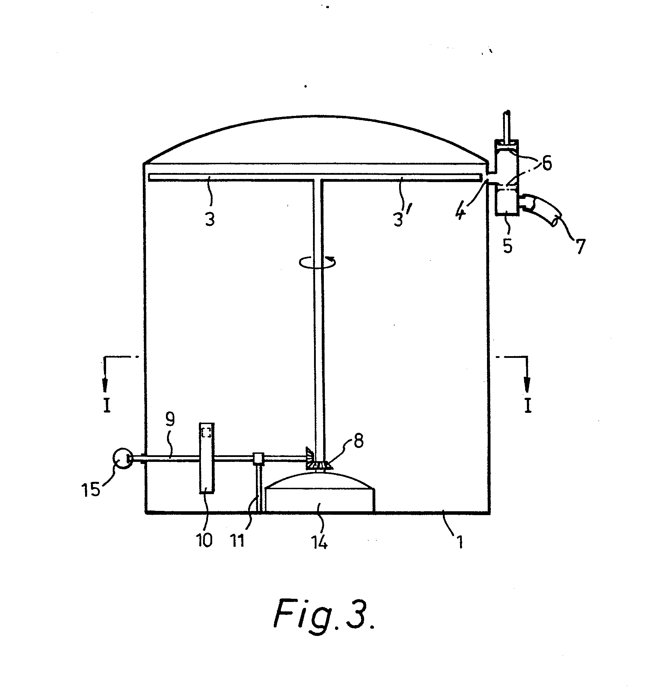

In the drawings, sealed cylindrical vessel 1 carried a central vertical rotatable shaft 2 bearing a pair of paddles 3, 3', which are shown in different rotative positions in Figs. 2 and 3. An opening 4 near the top of the cylindrical wall of the vessel (see Fig. 3) leads to a cylinder 5 containing a piston 6, which allows the interior of vessel 1 to communicate with an exit conduit 7 when in the raised position shown by the full lines but not when in the lower position shown dotted.

Vertical shaft 2 is driven through bevel gear 8 from horizontal shaft 9, which is itself rotated by Pelton wheel 10 and is supported at 11. Wheel 10 is rotated by incoming liquid or slurry entering by pipe 12 and leaves the wheel at 13 in a slightly downwards direction so that it is caused to circulate close to heating dome 14 and then to render the liquid in the vessel 1 turbulent. The arrows in Figs. 1 and 2 indicate generally the direction of flow.

A revolving paddle 16 disperses heated liquid rising from heating dome 14 and gently mixes digesting liquid.

In the Pelton wheel 10, the vanes are preferably hinged so that they stand up during the driving part of their cycle (i.e. in the enclosed annular space of the wheel) but are retracted in the non-driving non-enclosed part of their cycle.

Horizontal shaft 11 passes through a fluid-tight seal and bears an indicator disc 15, which shows whether or not the shaft is rotating.

The methane is removed by a suitable pipe (not shown) at the top of vessel 1.  Slurry is caused to enter pipe 12 by means of a pump (not shown) which is time controlled in association with piston 6.   When the pump is operative, the valve formed by 5, 6 and 7 is open.  Operation is otherwise as indicated above.

**0144213**

## CLAIMS

1. An air-tight vessel (1) for the anaerobic fermentation of a slurry of animal wastes to produce methane characterised in that one or more paddles (3, 3') is/are mounted on a vertical shaft (2), the paddles being rotatable in a horizontal plane at a level that will in operation coincide with or be just below the surface of the waste being fermented and also having a valve-controlled outlet (5, 6, 7) at approximately the same level as the paddles for material expelled mechanically by the paddles (3, 3'), an inlet (12) for fresh slurry, an outlet for methane gas and a means (14) for heating the slurry.

2. A vessel as claimed in Claim 1, in which the paddles (3, 3') are rotated by means of a Pelton wheel (10) situated towards the bottom of the vessel, the wheel being in a substantially vertical plane, being rotatable by the flow of incoming fresh slurry and connected to the paddles (3, 3') by suitable shafts and gearing (8, 9), and being positioned so that slurry, after passing through it, is directed at an angle slightly below the horizontal so as to come into close proximity to a heating dome (14) situated in the centre of the bottom of the vessel (1) and thereafter to agitate the slurry already in the vessel.

3. A vessel as claimed in Claim 2, including a pump to supply the fresh slurry and a timing control associated with the pump and the valve controlling the outlet for material expelled by the paddles, such that the valve is open when the pump is operating and closed when it is not.

4. A vessel as claimed in Claim 3, in which the timing control is such that the pump operates and the valve opens for 10 minutes; then the pump is inoperative and the valve closed for 30 minutes; with continuous repetition of the sequence.

5. A vessel as claimed in any preceding claim, including conduit means for the material expelled, the conduit terminating in a weir which opens to the atmosphere but prevents ingress of atmospheric air.

6.    A method of anaerobically fermenting a slurry of animal waste in an airtight vessel (1) to produce methane, characterised in that one or more paddles (3, 3') is/are rotated on the surface of the waste to break up any crust formed and expel the crust material through a conduit to atmosphere via a means (5, 6, 7) that will prevent air reaching the fermenting slurry.

7.    A method as claimed in Claim 6, in which the paddle(s) is/are rotated by a stream of incoming slurry acting on a Pelton wheel (10) connected to the paddle(s), the Pelton wheel diverting the stream to the proximity of a heater (14) and causing the stream to agitate the fermenting slurry.

8.    A method as claimed in Claim 7, in which there is a stream of incoming slurry only at times when the conduit to atmosphere is accessible to the fermenting slurry, the means for causing the stream to enter and permitting the crust material to leave being interlinked so that the paddle(s) rotate(s) only when more slurry is entering and the crust material can leave.

9.    A method as claimed in any one of Claims 6 to 8 in which the crust material is expelled via a weir.

0144213

III

14

2

16

II

8

16

II

9

III

10

1

*Fig.1.*

*Fig.2.*

3

3'

2

1

16

16

I

I

12

9

14

13

10

*Fig.3.*